Europäisches Patentamt

European Patent Office

Office européen des brevets

(11) Publication number: **0 124 665**
**B1**

(12) **EUROPEAN PATENT SPECIFICATION**

(45) Date of publication of patent specification: 20.04.88

(51) Int. Cl.⁴: **A 61 K 9/06,** A 61 K 35/04

(21) Application number: **83302533.1**

(22) Date of filing: **05.05.83**

(54) Dermatological compositions.

(43) Date of publication of application:
**14.11.84 Bulletin 84/46**

(45) Publication of the grant of the patent:
**20.04.88 Bulletin 88/16**

(84) Designated Contracting States:
**AT BE CH DE FR IT LI LU NL SE**

(56) References cited:
**WO-A-80/00660**
**GB-A-1 279 294**
**US-A-4 102 995**

(73) Proprietor: **QUINODERM LIMITED**
**Manchester Road Hollinwood**
**Oldham Lancashire OL8 4PB (GB)**

(72) Inventor: **Brown, John**
**90 Oakdale Drive**
**Heald Green Cheadle Cheshire SK8 3SW (GB)**

(74) Representative: **Quest, Barry et al**
**M'CAW & Co. 41-51 Royal Exchange Cross**
**Street**
**Manchester M2 7BD (GB)**

Courier Press, Leamington Spa, England.

# 0 124 665

**Description**

This invention relates to dermatological compositions particularly although not exclusively for human therapeutic use.

Crude tars such as coal and wood tar are effective dermatological therapeutic agents. However, known compositions containing crude tars have a heavy, greasy base and are not therefore wholly cosmetically acceptable.

An object of the present invention therefore is to provide a dermatological composition combining efficacy with cosmetic acceptability.

According to the invention therefore there is provided a dermatological composition comprising coal tar solution and/or wood tar and a base, characterised in that the said base consists solely of water, a gelling agent and a light emollient which is a liquid having emollient and solvent properties whereby the composition is in the form of a thixotropic gel comprising a stable gel emulsion of tar oils in water.

With this composition the active ingredient can be readily and conveniently applied in intimate contact with the skin such as to give effective dermatological action yet at the same time the composition can be in the form of a cosmetically acceptable vanishing preparation.

The invention permits use of coal tar and/or wood tar in their highly effective crude form without necessitating incorporation in a messy, greasy, cosmetically unacceptable formulation.

The gelling agent may be any suitable natural and/or synthetic substance although preferably a cellulose derivative is used.

The light emollient may be any suitable substance having both emollient and solvent properties which is capable of assisting in maintaining a stable gel emulsion of tar oils in water.

The invention will now be described further in the following Example.

A dermatological composition is prepared by mixing active ingredients with a light base.

The active ingredients comprise:

Strong coal tar solution BP (more specifically liquor picis carbonis fortis BPC which is a solution in 95% W. W. ethyl alcohol of coal tar fractions derived from coal distillation); and wood tar BP (which comprises fractions derived from the distillation of wood).

The light base comprises:

Water; a cellulose gelling agent (preferably the cellulose derivative sold by Courtaulds Limited under the trade name Celacol® Gum); and an emollient/solvent such as that sold by Atlas Chemical Industries UK Limited under the name Arlamol® E (which is a polyoxypropylene stearyl ether).

The constituents may be mixed in the following proportions:

| | |
|---|---|
| Strong coal tar solution , | —sufficient to give 2% by weight coal tar BP in the final mixture |
| Wood tar BP | —5% by weight |
| Arlamol® E | —5% by weight |
| Celacol® Gum (grade HPM 5000 GP) | —3.5% by weight |
| Water | —to 100% by weight. |

The resulting mixture is a thixotropic gel which spreads easily and cleanly on the skin and has "vanishing" properties. The composition is therefore cosmetically acceptable and also highly effective since intimate contact can be readily attained between the active ingredients (i.e. the tars) and the skin. The composition may be used in the treatment of Psoriasis and dermatitis in the chronic phase and has antipruritic, keratolytic and antiseptic properties as well as possible antiproliferative action in the case of Psoriasis.

It is of course to be understood that the invention is not intended to be restricted to the details of the above Example.

Thus for example strong coal tar BP may be used as an alternative to the BPC material. Also, if desired an antifungicide and preservative may be added such as Myacide® S.P. in a proportion of say 0.1% by weight.

The ingredients used in the Example are preferably mixed cold with the water being added last.

**Claims for the Contracting States: BE, CH, DE, FR, IT, LI, LU, NL, SE**

1. A dermatological composition comprising coal tar solution and/or wood tar and a base, characterised in that the said base consists solely of water, a gelling agent and a light emollient which is a liquid having emollient and solvent properties whereby the composition is in the form of a thixotropic gel comprising a stable gel emulsion of tar oils in water.

2. A composition according to claim 1, wherein said gelling agent comprises a cellulose derivative.

3. A composition according to claim 1 or 2, wherein said light emollient is a polyoxypropylene stearyl ether.

**0 124 665**

**Claims for Contracting State: AT**

1. A method of making a dermatological composition comprising mixing coal tar solution and/or wood tar and a base, characterised in that the said base is made by mixing together solely water, a gelling agent and a light emollient which is a liquid having emollient and solvent properties whereby the composition is in the form of a thixotropic gel comprising a stable gel emulsion of tar oils in water.

2. A method according to claim 1, wherein said gelling agent comprises a cellulose derivative.

3. A method according to claim 1 or 2, wherein said light emollient is a polyoxypropylene stearyl ether.

**Patentansprüche für die Vertragsstaaten: BE, CH, DE, FR, IT, LI, LU, NL, SE**

1. Dermatologisches Präparat, das aus einer Kohlenteer-Lösung und/oder Holzteer sowie einem Grundstoff besteht, dadurch gekennzeichnet, daß der genannte Grundstoff lediglich aus Wasser, einem Gelierstoff und einem leichten Linderungsmittel, das eine Flüssigkeit mit beruhigenden und löslichen Eigenschaften ist, besteht, wodurch das Präparat die Form eines thixotropischen Gels aus einer stabilen Gelemulsion von Teerölen in Wasser erhält.

2. Präparat entsprechend Anspruch 1, bei dem der genannte Gelierstoff ein Zellulosederivat ist.

3. Präparat entsprechend den Ansprüchen 1 oder 2, bei dem das leichte Linderungsmittel ein Polyoxypropylän-Stearylether ist.

**Patentansprüche für der Vertragsstaat: AT**

1. Verfahren zur Herstellung eines dermatologischen Präparats aus einer Kohlenteer-Lösung und/oder Holzteer sowie einem Grundstoff, dadurch gekennzeichnet, daß der genannte Grundstoff lediglich aus Wasser, einem Gelierstoff und einem leichten Linderungsmittel, das eine Flüssigkeit mit beruhigenden und löslichen Eigenschaften ist, besteht, wodurch das Präparat die Form eines thixotropischen Gels aus einer stabilen Gelemulsion von Teerölen in Wasser erhält.

2. Verfahren entsprechend Anspruch 1, bei dem der genannte Gelierstoff ein Zellulosederivat ist.

3. Verfahren entsprechend den Ansprüchen 1 oder 2, bei dem das leichte Linderungsmittel ein Polyoxypropylän-Stearylether ist.

**Revendications pour les États Contractants: BE, CH, DE, FR, IT, LI, LU, NL, SE**

1. Composition dermatologique comprenant une solution de goudron de houille et/ou du goudron de bois et une base, caractérisée en ce que ladite base consiste seulement en eau, un agent de gélification et un émollient léger qui est un liquide ayant des propriétés émollientes et dissolvantes, la composition étant sous forme d'un gel thixotrope comprenant une émulsion sous forme de gel stable d'huiles de goudron dans l'eau.

2. Composition selon la revendication 1, dans laquelle ledit agent de gélification comprend un dérivé cellulosique.

3. Composition selon la revendication 1 ou 2, dans laquelle ledit émollient léger est un éther polyoxypropylène stéaryle.

**Revendications pour l'état Contractant: AT**

1. Procédé de fabrication d'une composition dermatologique comprenant le mélangeage d'une solution de goudron de houille et/ou de goudron de bois et d'une base, caractérisée en ce que ladite base est faite en mélangeant seulement de l'eau, un agent de gélification et un émollient léger qui est un liquide ayant des propriétés émollientes et dissolvantes, la composition étant sous forme d'un gel thixotrope comprenant une émulsion sous forme de gel stable d'huiles de goudron dans l'eau.

2. Procédé selon la revendication 1, dans laquelle ledit agent de gélification comprend un dérivé cellulosique.

3. Procédé selon la revendication 1 ou 2, dans laquelle ledit émollient léger est un éther polyoxypropylène stéaryle.